# EUROPEAN PATENT SPECIFICATION

(11) **EP 2 403 568 B1**
(45) Date of publication and mention of the grant of the patent: **17.12.2014**
(21) Application number: 10706266.3
(22) Date of filing: 04.03.2010
(51) Int. Cl.: A61M 5/32

(54) **NEEDLE UNIT**
NADELEINHEIT
ENSEMBLE D'AIGUILLE

(30) Priority: 05.03.2009 EP 09003178
(43) Date of publication of application: 11.01.2012
(73) Proprietor: Sanofi-Aventis Deutschland GmbH, 65929 Frankfurt am Main (DE)
(72) Inventor: HEALD, Michael, Bergshire SL67SG (GB); EKMAN, Matthew, Cheshire SK101RD (GB)
(86) International application number: PCT/EP2010/052788
(87) International publication number: WO 2010/100243

(56) References cited:
- WO-A1-98/19723
- US-A- 5 971 964
- US-A1- 2003 105 430

## Description

This disclosure relates to a needle unit for a drug delivery device comprising a needle which is retractable into the drug delivery device from a starting position to an end position as well as a needle retainer having a stop member.

Drug delivery injection devices utilizing a hollow needle such as manual syringes, autoinjectors and pen type injectors introduce the risk of accidental needlestick injuries from a used and therefore potentially contaminated needle which may lead to the transfer of harmful diseases such as Hepatitis and HIV/AIDS. Therefore there is a need to provide disposable drug delivery devices wherein the needle can be made safe after use for example by retracting the contaminated needle into the device.

Document WO 2009/003234 A1 shows a syringe with a needle retaining system comprising a retractable needle, a needle seal, a retaining member and an ejector member, which is operable to release the retractable needle from the retaining member. The syringe furthermore comprises a plunger seal capable of engaging with the retractable needle and locking systems preventing re-use of the syringe after the needle has been retracted, wherein the locking systems prevent withdrawal of the plunger to extract the retracted needle.

Document WO 2006/119570 A1 shows a syringe comprising a plunger and a needle, which is mounted to a retractable needle mount. The needle mount can be engaged with the plunger which retracts the needle mount and hence the needle, which is mounted to the needle mount, into the syringe.

Document WO 98/19723 A1 shows a needle unit according to the preamble of claim 1.

It is an aim of the present invention to provide a needle unit for a drug delivery device which enhances the safety for a user.

This aim might be achieved by a needle unit according to the independent claim. Further features are subject matters of the dependent claims.

According to one aspect a needle unit for a drug delivery device is provided comprising a needle which is retractable into the drug delivery device from a starting position to an end position and a needle retainer having a stop member. The stop member is configured to prevent a re-exposure of the needle when the needle has been retracted into the drug delivery device.

The needle is part of a needle assembly which is formed as a needle having a needle mount. The needle mount may be a plastic coating covering the proximal end of the needle.

The drug delivery device is suitable to deliver a drug, in one embodiment the drug is expelled through a needle. Examples of drug delivery devices are pen-type injection devices, auto-injectors or syringes, for example disposable pre-filled syringes.

The drug delivery device may comprise a needle retainer. The needle retainer is fixed in its position with respect to
a housing, for example by means of mechanical friction or snapping means. Alternatively, the needle retainer might be glued to the housing. The needle retainer is configured to releasably engange the needle in a predetermined position with respect to the needle retainer when delivering the dose of the drug. After having delivered the dose the needle retainer releases the needle from an initial locked, restrained state to an unlocked state such that it can be retracted by retraction means.

In the injection device described in this document, during drug delivery the needle is secured in the needle retainer in a starting position. After unlocking the needle from the needle retainer and then retracting it completely into the drug delivery device the (used) needle is positioned in an end position within the housing of the device. The needle is retracted along a substantially longitudinal axis.

The needle retainer comprises blocking features, i.e. the stop member, such that on withdrawal of the needle an aperture, through which the needle protrudes, is closed preventing subsequent re-exposure.

The stop member is part of the needle retainer. In one embodiment the stop member limits the re-movement of the needle along the longitudinal axis by blocking the distal re-movement of the needle. In another embodiment, the stop member prevents any movement of the needle from the end position to the starting position, i.e. in distal direction with respect to the drug delivery device, once the needle has been retracted into the device. Hence re-exposure of the retracted needle is prevented.

According to one embodiment, the starting position of the needle is the position, where the needle is exposed and the end position is the position where the needle is retracted.

In one embodiment, the needle, which is positioned in the starting position, is configured to force the stop member in an radial outward direction with respect to said needle.

As long as the needle is positioned in the starting position, i.e. the needle is secured to the needle retainer when delivering the dose of the drug, the needle pushes the stop member radially outwardly. Preferably, the stop member may comprise a biasing part, for example a spring, so that it can be pushed outwardly by the needle.

According to one embodiment of the invention the stop member is configured to move to a position blocking an aperture through which the needle is retractable into the drug delivery device.

In one embodiment, the aperture is formed as an axial hole within the needle retainer, which is designed to guide the needle from the starting to the end position. In one embodiment, the aperture might also guide the needle from the end position to the starting position, i.e. when initially engaging the needle with the drug delivery device during manufacture and assembly of the drug delivery device.

After delivering the dose of the drug and after retracting the needle into the drug delivery device, the stop member can no longer be kept in its radial outward position by the presence of the needle. Therefore, the stop member moves radially towards the location vacated by the needle and hence blocks the aperture of the needle retainer preventing a re-exposure of the retracted needle, as the needle can no longer be moved through the aperture of the needle retainer in the distal direction with respect to the drug delivery device.

Before delivering the dose of the drug the needle must be arranged in the starting position, thus, it must be secured to the needle retainer. Therefore, in one embodiment, the stop member should be configured to allow the movement of the needle from the end position to the starting position to fix the needle into the needle retainer before the dose is delivered, i.e. when assembling the drug delivery device for example during its manufacture.

In one embodiment the stop member is configured to permit a movement of the needle into distal direction with respect to the aperture to the starting position while engaging the needle with said drug delivery device, e.g. during manufacturing and assembly of the drug delivery device.

According to another preferred embodiment the stop member comprises a spring arm, which is suitable to be pushed radially outwardly with respect to the needle.

In one embodiment, the stop member has a biasing part so that it can be pushed radially outwardly with respect to the needle when the needle is positioned in the starting position.

In another embodiment, the biasing part is a spring element, which is stressed when the needle is positioned in the starting position and which is decompressed when the needle is retracted into the drug delivery device.

In one embodiment the stop member comprises a clip. The stop member may comprise a flexible or biasing part so that the needle can push it radially outwardly when the needle is in the starting position. The flexible or biasing part might be formed as a flexible clip or clamp, which gets buckled or bowed when the needle pushes the stop member radially outwardly. When the needle has been retracted, said clip or clamp gets relaxed. Consequently, the stop member moves radially inwardly with respect to the (retracted) needle and hence the stop member blocks the aperture of the needle retainer so that the needle cannot be re-exposed.

According to one embodiment the drug delivery device is a syringe.

According to one embodiment the drug delivery device is a pen-type injection device.

According to another embodiment the device is a pen injector safety needle.

According to another aspect a drug delivery device is provided comprising a housing, a needle retainer, needle retraction means and a needle assembly, where the needle assembly is retractable into the housing from a starting position, where the needle is exposed, to an end position where the needle is not exposed. The needle retainer releases the needle assembly from an initial locked, restrained state to a free state such that it can be retracted by the needle retraction means and also provides a stop member such that on withdrawal of the needle the aperture through which the needle protrudes is closed preventing subsequent re-exposure.

In one embodiment the drug delivery device comprises a medicament. The medicament could be pre-filled in a cartridge or, if the drug delivery device is designed as a syringe, pre-filled in the syringe.

The term "medicament", as used herein, means a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,

wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,

wherein in a further embodiment the pharmaceutically active compound comprises at least one peptide for the treatment and/or prophylaxis of diabetes mellitus or complications associated with diabetes mellitus such as diabetic retinopathy,

wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane such as hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Further features and refinements become apparent from the following description of the exemplary embodiments in connection with the accompanying figures.
Figure 1 schematically shows a drug delivery device comprising a needle unit according to the present invention.
Figure 2A schematically shows a perspective view of the needle unit of Figure 1.
Figure 2B schematically shows the needle unit of Figure 2A wherein the needle is retracted.
Figure 3A schematically shows a proximal end view of the needle unit of Figures 1 to 2B with the needle in the starting position.
Figure 3B schematically shows a side view of the needle unit of Figure 3A.
Figure 4A schematically shows a proximal end view of the needle unit of Figures 1 to 3A wherein the needle is retracted.
Figure 4B schematically shows a side view of the needle unit of Figure 4A.
Figure 5A schematically shows a further embodiment of a needle unit.
Figure 5B shows the needle unit of Figure 5A wherein the needle is retracted.

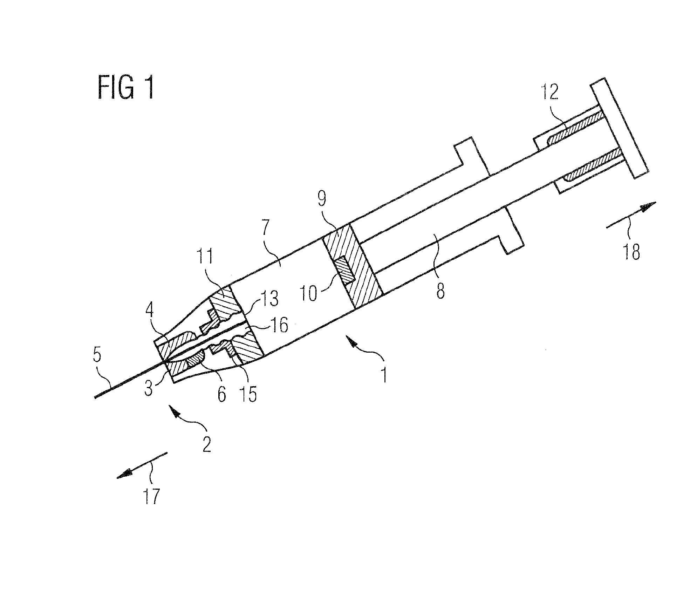

Figure 1 shows a drug delivery device 1 comprising a needle unit 2. The needle assembly 5, 16 comprises a needle 5 and a needle mount 16 which is located at the proximal end of the needle 5, the needle 5 is fixed to the needle mount 16. The needle retainer 3 comprises needle retainer arms 4 and a stop member 6. The drug delivery device 1 further comprises a housing 7, a plunger 8 and a plunger seal 9. The plunger seal 9 contains engaging means 10. The device 1 comprises furthermore a needle seal 11, retraction means 12, engaging means 13 which are configured to engage with the engaging means 10 of the plunger seal 9 as well as an ejector member 15.

The drug delivery device 1 has a distal end and a proximal end indicated by the directional arrows 17 (distal) and 18 (proximal), respectively. The distal end refers to that end of the drug delivery device 1 which is closest to the dispensing end of the drug delivery device 1. The proximal end is that end of the drug delivery device 1 which is opposite to the dispensing end.

The drug delivery device 1 is designed as a disposable pre-filled safety syringe. The drug delivery device 1 may be intended to dispense fixed doses of a drug or variable (preferably user-settable) doses.

The drug delivery device 1 comprises the housing 7. If the drug delivery device 1 is a syringe, as shown in Figure 1, the housing 7 is shaped as a barrel. If the drug delivery device 1 is a pen-type injection device (not shown), the housing 7 might contain further elements, for example a holding member (not shown in Figure 1) containing a medicament or a drug container, for example a cartridge, wherein the dose of the drug is stored.

The barrel may be built from glass or plastic. The plunger 8 and the plunger seal 9 can move within the barrel along a substantially longitudinal axis.

The needle retainer 3 is fixed with respect to the housing 7 for example by means of mechanical friction or with engaging clips. The needle retainer 3 might also be glued to the housing 7. The needle retainer 3 secures the needle assembly 5, 16 by securing the needle mount 16 against displacement with respect to the needle retainer 3 before and when delivering the dose of the drug. The needle retainer 3 has two or more needle retainer arms 4.

The proximal part of the needle 5 is supported in the needle mount 16, which may increase the friction and facilitate engagement of the needle assembly 5, 16 with the plunger seal 9 for retracting the needle assembly 5, 16 into the drug delivery device 1, as explained later. The needle assembly 5, 16 is preferably secured within the needle retainer 3 by means of mechanical friction or by appropriate mechanical location features. Thereby, the needle retainer arms 4 engage with the needle assembly 5, 16, via the needle mount 16 which supports the needle 5. The needle retainer arms 4 may also comprise engaging means, for example a nib, for engaging with the needle mount 16. The needle retainer arms 4 may be made of a flexible material.

When the needle assembly 5, 16 is secured to the needle retainer 3 it is positioned in the starting position. When the needle assembly 5, 16 is retracted completely into the drug delivery device 1 after the dose of the drug has been delivered it has been moved to the end position.

The needle seal 11 is placed at the proximal end of the needle unit 2. The housing 7, needle seal 11 and plunger seal 9 form a fluid tight container for the drug. The needle seal 11 may be made of a resilient material, for example an elastomer, e.g. rubber, and provides a fluid seal between an internal surface of the housing 7 the needle retainer 3 and hence the distal opening of the housing 7, which means that the drug cannot move between the housing 7 and the needle assembly 5, 16 unless via an internal bore of the needle 5.
The needle seal 11 is releasably secured against displacement with respect to the housing 7 and is intended to move in the distal direction with respect to the housing 7 after the dose of the drug has been delivered. The needle seal 11 might be releasably engaged with the housing 7 by means of mechanical friction, by engaging clips or by means of a flange.

The plunger seal 9 is preferably made of resilient material such as an elastomer, e.g. rubber, providing a fluid tight seal between an internal surface of the housing 7, the plunger 8 and hence the proximal opening of the housing 7 which means that the drug cannot move between the housing 7 and the plunger 8. The plunger seal 9 might be integrally formed with the plunger 8. However, the plunger seal 9 and the plunger 8 might also be separately formed, i.e. the plunger seal 9 might be connected to the plunger 8.

The housing 7, plunger seal 9 and seal needle 11 form a fluid tight medicament container.

In this embodiment the stop member 6 is part of the needle retainer 3, as shown in Figure 1. Thereby, the stop member 6 might also extend radially outwardly beyond the needle retainer 3.

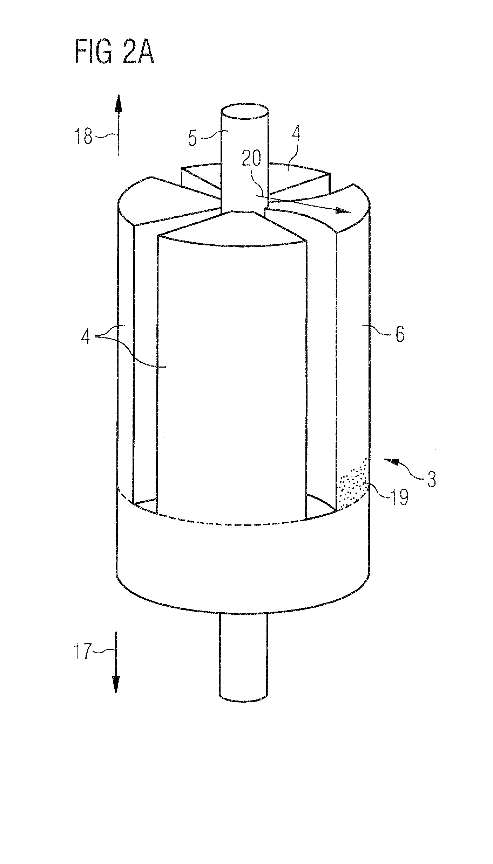

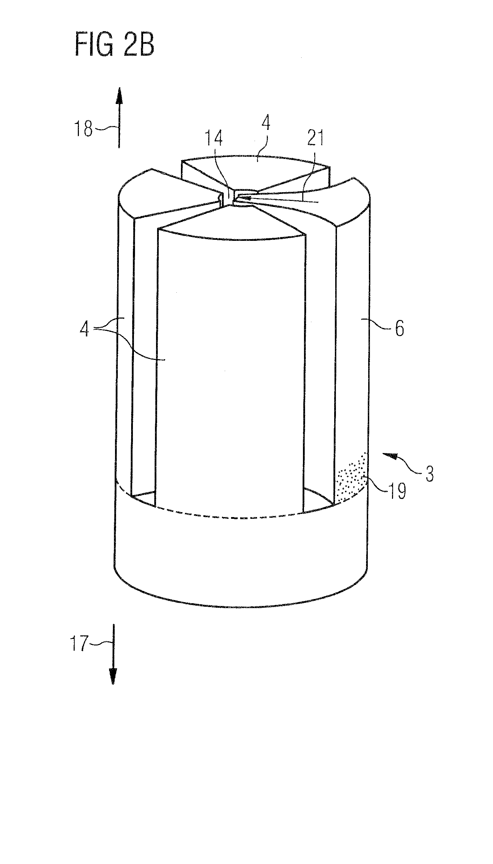

The stop member 6 preferably comprises a flexible or biasing part (see Figures 2A and 2B). Preferably, the flexible or biasing part is a spring arm, which is pre-stressed when the needle assembly 5, 16 is in the starting position and which is relaxed when the needle assembly 5, 16 is retracted into the drug delivery device 1. The flexible or biasing part might also be a flexible clip or clamp, which buckles or bows when the needle assembly 5, 16 is in the starting position. When the needle assembly 5, 16 is retracted the clip or clamp is relaxed.

To deliver the dose of the drug the user depresses the plunger 8, which in response moves in the distal direction with respect to the housing 7. The plunger seal 9 is also pushed distally with respect to the housing 7, towards the needle assembly 5, 16 and the needle retainer 3. This forces the drug out of the medicament or drug container, for example the cartridge, and at the end of the dose the distal end of the plunger seal 9 abuts the proximal end of the needle seal 11 after the content of the drug delivery device 1 has been completely dispensed. The user continues to depress the plunger 8 thus pushing the plunger seal 9, needle seal 11 and as a result the ejector member 15 in distal direction with respect to the housing 7 towards the needle assembly 5, 16 and the needle retainer 3.

The ejector member 15 is arranged to be moveable towards the needle retainer 3 and on contact and subsequent interaction displaces, in one embodiment, the needle retainer arms 4 radially outwards with respect to the needle assembly 5, 16. Hence, the needle assembly 5, 16 is unsecured from the needle retainer 3, i.e. the needle retainer arms 4 and in a next step the needle assembly 5, 16 can be retracted into the drug delivery device 1.

In one embodiment the ejector member 15 displaces the needle retainer arms 4 as well as the stop member 6 to unlock the needle assembly 5, 16 from the needle retainer 3. In another embodiment - as explained previously - the ejector member 15 displaces only the needle retainer arms 4 and the needle assembly 5, 16 may slide along the stop member 6 in proximal direction with respect to the housing 7 once unlocked from the needle retainer arms 4 and retracted.

For retracting the needle assembly 5, 16 into the drug delivery device 1 the plunger seal 9 comprises the engaging means 10 which are suitable to engage with the mating engaging means 13 of the needle assembly 5, 16, i.e. the proximal end of the needle mount 16. Thereby, the engaging means 10 might comprise a lug and the mating engaging means 13 might comprise a notch or vice versa.

In this embodiment the engaging means 10 comprise a notch. When the proximal end of the needle mount 16 is no longer covered by the needle seal 11, i.e. the needle seal 11 is pushed in distal direction by the plunger seal 9, the notch fits to the mating engaging means 13 of the needle assembly 5, 16, so that the proximal end of the needle mount 16 engages with the notch by means of mechanical friction.

When the needle assembly 5, 16 is positioned in the starting position it imparts a radially outward force to the stop member 6. The biasing or flexible part of the stop member 6 is hence in a pre-stressed state and the stop member 6 is kept in an radial outward position with respect to the needle assembly 5, 16.

For assembly, for example while manufacturing the drug delivery device 1, the needle assembly 5, 16 might be moved axially in distal direction with respect to the housing 7 through an aperture 14 to the starting position. The aperture 14 is shown explicitly in Figures 2B to 5B. The aperture 14 may be formed as an axial hole within the needle retainer 3, and is designed to guide the needle assembly 5, 16 for example from the starting to the end position. In one embodiment, the stop member 6 permits a movement of the needle assembly 5, 16 into distal direction with respect to the needle retainer 3 to the starting position of the needle assembly 5, 16 while initially engaging the needle assembly 5, 16 with the drug delivery device 1 during assembly. In this embodiment, the needle assembly 5, 16 is inserted into the drug delivery device 1 during assembly of the device 1, is moved into distal direction with respect to the device 1 and is finally fixed into the needle retainer 3, i.e. between the needle retainer arms 4. Thereby, a distal movement of the needle assembly 5, 16 to the starting position is only allowed once - during assembly of the drug delivery device 1.

When the dose has been delivered the needle assembly 5, 16 is retracted into the housing 7. The retraction of the needle assembly 5, 16 is described later on in more detail. When the needle assembly 5, 16 is retracted, the stop member 6 relaxes to a position blocking the aperture 14 of the needle retainer 3 as the needle assembly 5, 16 can no longer maintain the stop member 6 in the radial outward position with respect to the needle assembly 5, 16. Hence, the stop member 6 moves radially inwardly with respect to the (retracted) needle assembly 5, 16 and blocks the aperture 14. Consequently, any subsequent attempt to move the needle assembly 5, 16 in a distal direction through the aperture 14 is limited by the stop member 6, thus preventing re-exposure of the retracted needle assembly 5, 16.

For retracting the needle assembly 5, 16, the drug delivery device 1 comprises the retraction means 12, which might comprise a spring or a clip. After unlocking the needle assembly 5, 16 from the needle retainer 3, i.e. the needle retainer arms 4, the retraction means 12 might automatically pull the plunger 8, the plunger seal 9 and the needle assembly 5, 16, which is engaged with the plunger seal 9, proximally into the housing 7.

For this purpose, the initially compressed retraction means 12 must decompress so that the plunger 8 moves to proximal direction with respect to the housing 7. Proximal movement may be achieved by decompression of the retraction means 12, for example a spring, when an engagement arm of the plunger 8 reaches the proximal end of the housing 7. Due to decompression the plunger 8 is moved in the proximal direction with respect to the housing 7, thereby retracting the plunger seal 9 and the needle assembly 5, 16 coupled thereto proximally. Hence, a re-use of the drug delivery device 1 is prevented and a safe disposal of the device 1 is possible. One embodiment of a retraction means 12 is for example described in document WO 2009/003234 A1.

In an alternative embodiment the user manually retracts the needle assembly 5, 16 by pulling proximally the plunger 8 after pushing distally the plunger 8 in order to dispense the drug.

Figure 2A schematically shows a perspective view of the needle unit of Figure 1. The same reference numerals apply for the description of Figure 2A as for the description of Figure 1.

Figure 2A shows the needle retainer 3 with three needle retainer arms 4. The needle assembly 5, 16 is secured within the arms 4 of the needle retainer 3, preferably by means of mechanical friction. However, it can also be secured between the needle retainer arms 4 by means of a lug or a protrusion engaging with the needle mount 16 (not shown in Figure 2A) of the needle assembly 5, 16. The needle assembly 5, 16 is positioned in the starting position. The needle retainer 3 comprises a stop member 6. The stop member 6 comprises a biasing part 19 (indicated by the dots in Figure 2A). The stop member 6 might have an angled shape, as shown in Figure 2A.

The needle assembly 5, 16 imparts a radial force to the stop member 6. The force is indicated by arrow 20. Hence, the stop member 6, in particularly the biasing part 19 of the stop member 6, is pre-stressed so that the stop member 6 is held in a radial outward position with respect to the needle assembly 5, 16 so that the needle assembly 5, 16 can axially move (through the aperture 14, which is occupied by the needle assembly 5, 16 in Figure 2A) with respect to the housing 7 once the needle assembly 5, 16 is unlocked from the needle retainer 3. This enables retraction of the needle assembly 5, 16 into the drug delivery device 1 in a subsequent step.

As already explained the stop member 6 may comprise the biasing part 19, for example a spring or a clip so that it can be pushed radially outwardly by the needle assembly 5, 16. One embodiment of the stop member 6 comprises a hinge configured so that the stop member 6 blocks the aperture 14. This hinge can be integrally formed with the stop member 6. In one embodiment the stop member 6 is made of a flexible material configured so that the stop member 6 blocks the aperture 14. The stop member 6 is pushed radially outwardly when pushed onto by the needle assembly 5, 16.

In one embodiment, the stop member 6 may be part of the needle retainer 3, as indicated by Figure 2A. However, the stop member 6 may also be connected to the needle retainer 3.

Figure 2B schematically shows the needle unit of Figure 2A wherein the needle assembly is retracted. Hence, the needle assembly is not shown in Figure 2B. Figure 2B indicates the aperture 14 of the needle retainer 3.

In Figure 2B the needle assembly 5, 16 is retracted into the drug delivery device 1 (not shown). For example the needle assembly 5, 16 is retracted to the end position. Once the needle assembly 5, 16 is retracted it can no longer impart force on the stop member 6 which causes the stop member 6, in particular the biasing part 19 of the stop member 6, to decompress so that the stop member 6 moves radially inwardly with respect to the needle assembly 5, 16, which is indicated by the arrow 21. Hence, the stop member 6 blocks the aperture 14 and therefore prevents a subsequent movement of the needle assembly 5, 16 through the aperture 14 in distal direction with respect to the housing 7.

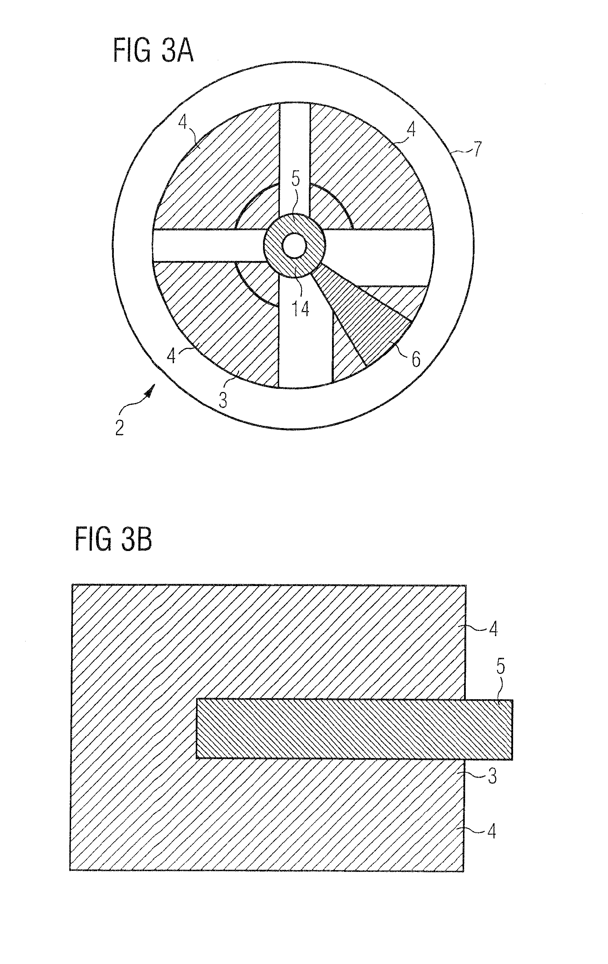

Figure 3A schematically shows a proximal end view of the needle unit of Figures 1 to 2B with the needle assembly in the starting position. The same reference numerals apply for the description of Figure 3A as for the description of Figure 1, 2A and 2B.

Figure 3A represents a top view of the proximal end of the needle unit 2, comprising the needle retainer 3 with the three needle retainer arms 4 and the needle assembly 5, 16. The needle assembly 5, 16 comprises the needle 5, which is covered by the needle mount 16. The needle retainer 3 comprises the stop member 6. Additionally, Figure 3A presents the aperture 14 of the needle retainer 3 which is filled by the needle assembly 5, 16. Figure 3A also shows the housing 7 of the drug delivery device 1 (indicated by the outer circle in Figure 3A).

The needle retainer 3 could be fixed in a variety of ways.
The needle retainer 3 is fixed with respect to the housing 7, for example by means of mechanical friction.

Figure 3A shows the needle assembly 5, 16 in the starting position before dispensing a dose of drug. The needle assembly 5, 16 is secured between the needle retainer arms 4 and the stop member 6 which prevent the displacement of the needle assembly 5, 16 with respect to the needle retainer 3. The needle assembly 5, 16 could be held within the needle retainer in a variety of ways. The needle assembly 5, 16 may be held within the needle retainer arms 4 by means of mechanical friction. The needle assembly 5, 16 is positioned in the aperture 14 of the needle retainer 3.

The stop member 6 preferably comprises a biasing or flexible part (see Figures 2A and 2B), for example the stop member 6 comprises a spring arm or a clip, as already explained previously. Preferably, the stop member 6 may be part of needle retainer 3 or may be connected to the needle retainer 3.

The needle assembly 5, 16 imparts a force on the stop member 6. Thereby, the biasing or flexible part of the stop member 6 gets pre-stressed and the stop member 6 is pushed radially outwardly, enabling an axial movement of the needle assembly 5, 16 with respect to the housing 7 and hence, a retraction of the needle assembly 5, 16 into the drug delivery device 1 once the needle assembly 5, 16 is unsecured from the needle retainer 3, as the aperture 14 is kept free from the stop member 6.

Figure 3B schematically shows a side view of the needle unit of Figure 3A.

Figure 3B represents a side view of the needle unit 2 of Figure 3A seen from the left side. Thereby, Figure 3B shows the needle retainer 3 with the needle retainer arms 4 engaging the needle assembly 5, 16, which is again in the starting position. The needle assembly 5, 16 blocks completely the aperture 14 of the needle retainer 3. Therefore, the stop member 6 (not shown), is pushed radially outwardly by the needle assembly 5, 16.

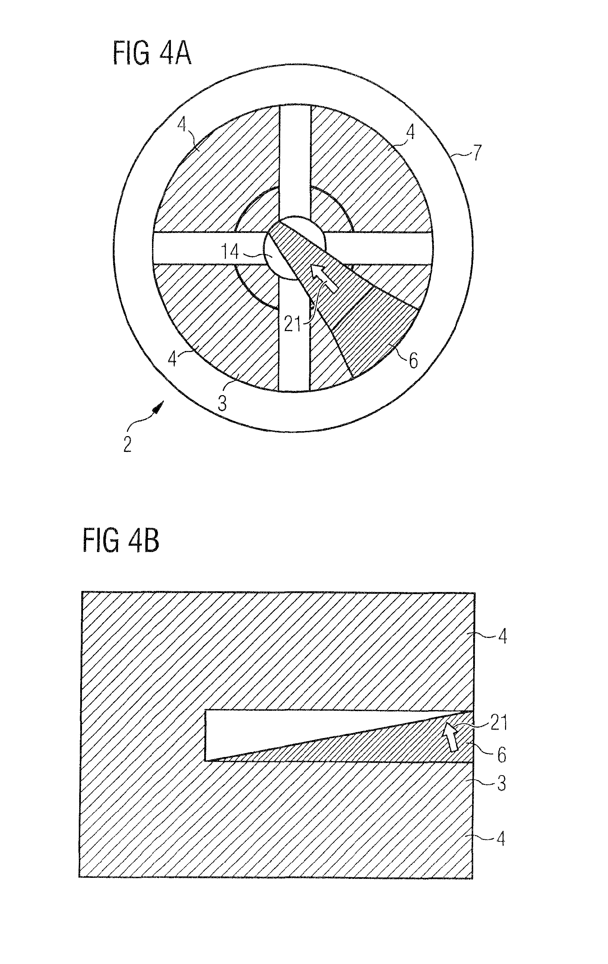

Figure 4A schematically shows a proximal end view of the needle unit of Figures 1 to 3A with the needle assembly in the end position. In contrast to Figure 3A the needle assembly 5, 16 is retracted in Figure 4A and hence, Figure 4A does not shown the needle assembly 5, 16.

Figure 4A shows the needle retainer 3 with three needle retainer arms 4. The needle retainer 3 comprises the stop member 6. Figure 4A also presents the aperture 14 of the needle retainer 3 and indicates the housing 7 of the drug delivery device (outer circle in Figure 4A).

Figure 4A does not show the needle assembly 5, 16, as the needle assembly 5, 16 is in a retracted position, for example the end position. When retracting the needle assembly 5, 16, the needle assembly 5, 16 sets free the aperture 14 of the needle retainer 3. Consequently, the needle assembly 5, 16 can no longer exert force on the stop member 6. Hence, the flexible or biasing part 19 of the stop member 6 (see Figures 2A and 2B) is decompressed and the stop member 6 relaxes radially inwardly to a position blocking the aperture 14 of the needle retainer 3, as it is indicated by the solid arrow 21. As the needle assembly 5, 16 has to be moved through the aperture 14 of the needle retainer 3 into distal direction with respect to the device 1 in order to be arranged in its starting position, a re-exposure of the needle assembly 5, 16 is consequently prevented.

Figure 4B schematically shows a side view of the needle unit of Figure 4A.

Figure 4B represents a side view of the needle unit 2 of Figure 4A seen from the left side. Figure 4B shows the needle retainer 3 with the needle retainer arms 4. In addition, Figure 4A shows the stop member 6 which has moved radially inwardly with respect to the (retracted) needle assembly 5, 16, as indicated by the solid arrow 21. Due to its movement in radial inward direction with respect to the (retracted) needle assembly 5, 16 the stop member 6 blocks the aperture 14 of the needle retainer 3 and a re-exposure of the retracted needle assembly 5, 16 is prevented.

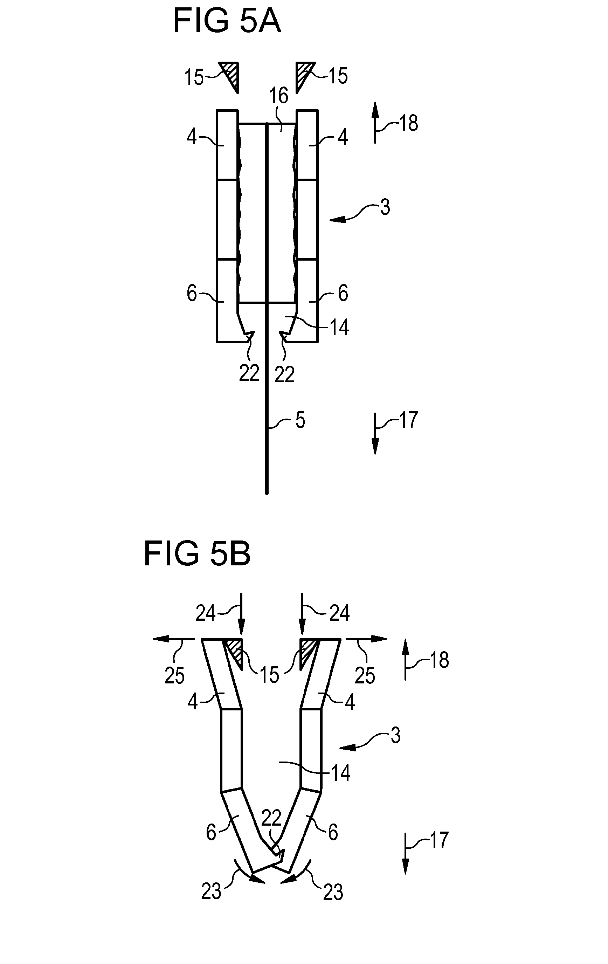

In a further embodiment shown in figures 5A and 5B the needle retainer 3 has additional arms pointing distally, which are forced radially outward by the presence of the needle assembly 5, 16. On removal of the needle assembly 5, 16 i.e. proximal retraction into the housing 7 of the device 1 the biased arms relax radially inward blocking the needle retainer aperture 14 and preventing subsequent passage of the needle assembly 5, 16 to a re-exposed position relative to the housing 7. Non return features at the most distal end of the distal facing needle retainer arms 4 further assist blocking subsequent distal travel of the needle assembly 5, 16.

Figure 5A schematically shows a further embodiment of a needle unit.

Figure 5A shows the needle retainer 3 which comprises in this embodiment four needle retainer arms 4 (for clarity reasons only two needle retainer arms 4 are shown in Figure 5A) located at the proximal end of the needle retainer 3. The needle assembly 5, 16, i.e. the needle 5 which is supported in the needle mount 16, is secured between the needle retainer arms 4, preferably by means of mechanical friction though the needle assembly 5, 16 could be secured by a variety of ways e.g. mechanical location or snap fits. Hence, the needle assembly 5, 16 is in the starting position. The needle assembly 5, 16 occupies the aperture 14 of the needle retainer 3.

The needle retainer 3 comprises the stop members 6. The stop members 6 comprise in this embodiment four arms, whereas for clarity reasons only two arms of stop member 6 are shown in Figure 5A. In this embodiment the stop members 6 are arranged at the distal end of the needle retainer 3. The stop members 6 comprise at the distal end coupling means 22, in this embodiment claws, which are arranged radially inwardly with respect to the needle assembly 5, 16 and which are configured to engage with each other after the needle assembly 5, 16 has been retracted into the drug delivery device (not shown in Figure 5A). These non-return features, i.e. the coupling means 22, at the most distal end of the needle retainer arms 4 further assist blocking subsequent distal travel of the needle assembly 5, 16 (see Figure 5B).

As already described in connection with Figures 1 to 4B, the stop members 6 preferably comprise a flexible or biasing part, for example a spring arm or a clip (see Figures 2A and 2B). In Figure 5A the needle assembly 5, 16 is in the starting position pushing the stop members 6, in particular the biasing part of the stop members 6, radially outwardly with respect to the needle assembly 5, 16.

Figure 5A also shows ejector member 15, which is arranged at the proximal end of the needle retainer 3 and which is operable to release the retractable needle assembly 5, 16 from the needle retainer 3, i.e. the needle retainer arms 4, after the content of the drug delivery device has been delivered, as described previously.

Figure 5B shows the needle unit of Figure 5A wherein the needle assembly is retracted.

After the dose has been delivered completely the needle seal 11 (not shown in Figure 5A) pushes onto the ejector member 15 which then moves in proximal direction towards the needle retainer 3, as indicated by arrow 24, displacing the needle retainer arms 4 radially outwardly (see arrow 25). Hence, the needle assembly 5, 16 is unsecured from the needle retainer 3, i.e. the needle retainer arms 4 and can be retracted into the drug delivery device, as explained in conjunction with the description of Figure 1.

Figure 5B shows the needle unit 2 after the needle assembly 5, 16 has been retracted into the device, hence the needle assembly 5, 16 is not shown in Figure 5B. As the needle assembly 5, 16 is retracted it can no longer impart force on the stop members 6. Consequently, the stop members 6, in particular the biasing part of the stop members 6, decompress moving radially inwardly with respect to the (retracted) needle assembly 5, 16, which is indicated by arrow 23. Thereby, the coupling means 22 of the stop members 6 engage with each other, preventing further movement of the stop members 6 in radial outward direction with respect to the (retracted) needle assembly 5, 16. Hence, the stop members 6 block effectively the aperture 14 preventing movement of the needle assembly 5, 16 through the aperture 14 in distal direction.

Other implementations are within the scope of the following claims. Elements of different implementations may be combined to form implementations not specifically described herein.

### Reference Numerals

- 1: Drug delivery device
- 2: Needle unit
- 3: Needle retainer
- 4: Needle retainer arms
- 5: Needle
- 6: Stop member
- 7: Housing
- 8: Plunger
- 9: Plunger seal
- 10: Engaging means
- 11: Needle seal
- 12: Retraction means
- 13: Engaging means
- 14: Aperture
- 15: Ejector member
- 16: Needle mount
- 17: Distal end
- 18: Proximal end
- 19: Biasing part
- 20: Arrow
- 21: Arrow
- 22: Coupling means
- 23: Arrow
- 24: Arrow
- 25: Arrow

## Claims

1. A needle unit (2) for a drug delivery device (1),
wherein the needle unit (2) comprises a needle (5) which is retractable into the drug delivery device (1) from a starting position to an end position and a needle retainer (3) having a stop member (6), wherein the stop member (6) is part of the needle retainer (3), and wherein the stop member (6) is configured to prevent a re-exposure of the needle (5) when the needle (5) has been retracted into the drug delivery device (1), wherein the needle (5) is part of a needle assembly (5, 16) which is formed as a needle (5) having a needle mount (16) and wherein the needle retainer (3) secures the needle assembly (5, 16) by securing the needle mount (16) against displacement with respect to the needle retainer (3) before and when delivering a dose of a drug,
**characterized in that**
the needle mount (16) is a plastic coating covering a proximal end of the needle (5), wherein the needle retainer (3) comprises needle retainer arms (4), and wherein the needle retainer arms (4) engage with the needle assembly (5, 16) via the needle mount (16) which supports the needle (5), and wherein the stop member (6) is configured to move to a position blocking an aperture (14) through which the needle (5) is retractable into the drug delivery device (1).

2. The needle unit (2) according to claim 1,
wherein the starting position of the needle (5) is the position, where the needle (5) is exposed and the end position is the position where the needle (5) is retracted.

3. The needle unit (2) according to any of the previous claims,
wherein the needle retainer (3) is fixed in its position with respect to a housing (7).

4. The needle unit (2) according to any of the previous claims,
wherein the needle retainer (3) is configured to release the needle (5) from a locked state to an unlocked state.

5. The needle unit (2) according to claim 4,
wherein retraction means (12) are configured to retract the needle (5) when the needle (5) is unlocked.

6. The needle unit (2) according to any of the previous claims,
wherein the needle (5), which is positioned in the starting position, is configured to force the stop member (6) in a radial outward direction with respect to said needle (5).

7. The needle unit (2) according to any of the previous claims,
wherein the stop member (6) comprises a hinge.

8. The needle unit (2) according to claim any of the previous claims,
wherein the stop member (6) is configured to permit a movement of the needle (5) into distal direction with respect to the aperture (14) to the starting position while engaging the needle (5) with said drug delivery device (1).

9. The needle unit (2) according to any of the claims 1 to 8,
wherein the stop member (6) comprises a clip.

10. A drug delivery device (1) comprising a needle unit (2) according to any of the previous claims and further comprising a housing (7), the needle retainer (3), needle retraction means (12) and the needle assembly (5, 16) comprising a needle (5), wherein the needle assembly (5, 16) is retractable into the housing (7) from a starting position, where the needle (5) of the needle assembly (5, 16) is exposed, to an end position, where the needle (5) is not exposed, and wherein the needle retainer (3) is configured to release the needle assembly (5, 16) from an initial locked state to an unlocked state such that it can be retracted by the needle retraction means (12) and also provides the stop member (6) such that on withdrawal of the needle (5) an aperture (14) through which the needle (5) protrudes is at least partly closed preventing subsequent re-exposure of the needle (5).

11. Drug delivery device (1) according to claim 10,
comprising an ejector member (15) being configured to unlock the needle assembly (5, 16).

12. Drug delivery device (1) according to any of claims 10 or 11,
comprising a needle seal (11) being placed at a proximal end of the needle unit (2).

## Patentansprüche

1. Nadeleinheit (2) für eine Arzneimittelabgabevorrichtung (1),
worin die Nadeleinheit (2) eine Nadel (5), die aus einer Ausgangsstellung in eine Endstellung in die Arzneimittelabgabevorrichtung (1) zurückziehbar ist, und einen Nadelhalter (3) mit einem Anschlagelement (6) umfasst, worin das Anschlagelement (6) Teil des Nadelhalters (3) ist, und worin das Anschlagelement (6) ausgelegt ist, eine erneute Exposition der Nadel (5) zu verhindern, wenn die Nadel (5) in die Arzneimittelabgabevorrichtung (1) zurückgezogen worden ist, worin die Nadel (5) Teil einer Nadelanordnung (5, 16) ist, die als Nadel (5) mit einer Nadelhalterung (16) geformt ist, und worin der Nadelhalter (3) die Nadelanordnung (5, 16) fixiert, indem er die Nadelhalterung (16) gegen eine Verschiebung bezüglich des Nadelhalters (3) vor und bei Abgabe einer Dosis eines Arzneimittels sichert,
**dadurch gekennzeichnet, dass**
die Nadelhalterung (16) eine Kunststoffbeschichtung ist, die ein proximales Ende der Nadel (5) bedeckt, worin der Nadelhalter (3) Nadelhalterarme (4) umfasst und worin die Nadelhalterarme (4) mit der Nadelanordnung (5, 16) über die Nadelhalterung (16), welche die Nadel (5) stützt, in Eingriff steht, und worin das Anschlagelement (6) ausgelegt ist, sich in eine Stellung zu bewegen, die eine Öffnung (14) blockiert, durch welche die Nadel (5) in die Arzneimittelabgabevorrichtung (1) zurückziehbar ist.

2. Nadeleinheit (2) nach Anspruch 1,
worin die Ausgangsstellung der Nadel (5) die Stellung ist, in der die Nadel (5) freiliegt und worin die Endstellung die Stellung ist, in der die Nadel (5) zurückgezogen ist.

3. Nadeleinheit (2) nach einem der vorhergehenden Ansprüche,
worin der Nadelhalter (3) in seiner Stellung bezüglich eines Gehäuses (7) fixiert ist.

4. Nadeleinheit (2) nach einem der vorhergehenden Ansprüche,
worin der Nadelhalter (3) ausgelegt ist, die Nadel (5) aus einem arretierten Zustand in einen nicht arretierten Zustand freizugeben.

5. Nadeleinheit (2) nach Anspruch 4,
worin Rückzugsmittel (12) ausgelegt sind, die Nadel (5) zurückzuziehen, wenn die Nadel (5) nicht arretiert ist.

6. Nadeleinheit (2) nach einem der vorhergehenden Ansprüche,
worin die Nadel (5), die in der Ausgangsstellung positioniert ist, ausgelegt ist, das Anschlagelement (6) in eine radiale Auswärtsrichtung bezüglich der Nadel (5) zu drücken.

7. Nadeleinheit (2) nach einem der vorhergehenden Ansprüche,
worin das Anschlagelement (6) ein Scharnier umfasst.

8. Nadeleinheit (2) nach einem der vorhergehenden Ansprüche,
worin das Anschlagelement (6) ausgelegt ist, eine Bewegung der Nadel (5) in die distale Richtung bezüglich der Öffnung (14) in die Ausgangsstellung zu gestatten, während die Nadel (5) mit der Arzneimittelabgabevorrichtung (1) in Eingriff gebracht wird.

9. Nadeleinheit (2) nach einem der Ansprüche 1 bis 8,
worin das Anschlagelement (6) eine Klammer umfasst.

10. Arzneimittelabgabevorrichtung (1), umfassend eine Nadeleinheit (2) nach einem der vorherigen Ansprüche und ferner umfassend ein Gehäuse (7), den Nadelhalter (3), Nadelrückzugsmittel (12) und die eine Nadel (5) umfassende Nadelanordnung (5, 16), worin die Nadelanordnung (5, 16) aus einer Ausgangsstellung, in der die Nadel (5) der Nadelanordnung (5, 16) freiliegt, in eine Endstellung, in der die Nadel (5) nicht freiliegt, in das Gehäuse (7) zurückgezogen werden kann, und worin der Nadelhalter (3) ausgelegt ist, die Nadelanordnung (5, 16) aus einem anfänglichen arretierten Zustand in einen nicht arretierten Zustand freizugeben, so dass sie von den Nadelrückzugsmitteln (12) zurückgezogen werden kann, und auch das Anschlagelement (6) bereitstellt, so dass beim Zurückziehen der Nadel (5) eine Öffnung (14), durch welche die Nadel (5) vorsteht, zumindest teilweise verschlossen wird, wodurch eine erneute Exposition der Nadel (5) verhindert wird.

11. Arzneimittelabgabevorrichtung (1) nach Anspruch 10,
umfassend ein Auswurfelement (15), das ausgelegt ist, die Nadelanordnung (5, 16) aus ihrer Arretierung zu lösen.

12. Arzneimittelabgabevorrichtung (1) nach einem der Ansprüche 10 oder 11, umfassend eine Nadeldichtung (11), die an einem proximalen Ende der Nadeleinheit (2) platziert wird.

## Revendications

1. Unité d'aiguille (2) pour un dispositif d'administration de médicament (1), dans laquelle l'unité d'aiguille (2) comprend une aiguille (5) qui est rétractable dans le dispositif d'administration de médicament (1) depuis une position de départ jusqu'à une position d'extrémité et un dispositif de retenue d'aiguille (3) ayant un organe d'arrêt (6), dans laquelle l'organe d'arrêt (6) fait partie du dispositif de retenue d'aiguille (3) et dans laquelle l'organe d'arrêt (6) est configuré pour empêcher une réexposition de l'aiguille (5) lorsque l'aiguille (5) a été rétractée dans le dispositif d'administration de médicament (1), dans laquelle l'aiguille (5) fait partie d'un ensemble d'aiguille (5, 16) qui est formé en tant qu'aiguille (5) ayant un support d'aiguille (16), et dans laquelle le dispositif de retenue d'aiguille (3) fixe l'ensemble d'aiguille (5, 16) en fixant le support d'aiguille (16) pour empêcher le déplacement par rapport au dispositif de retenue d'aiguille (3) avant et au cours de l'administration d'une dose de médicament,
**caractérisée en ce que**
le support d'aiguille (16) est un revêtement plastique couvrant une extrémité proximale de l'aiguille (5), le dispositif de retenue d'aiguille (3) comprenant des bras de retenue d'aiguille (4), et les bras de retenue d'aiguille (4) s'engageant avec l'ensemble d'aiguille (5, 16) par le biais du support d'aiguille (16) qui supporte l'aiguille (5), et l'organe d'arrêt (6) étant configuré pour se déplacer dans une position bloquant une ouverture (14) à travers laquelle l'aiguille (5) est rétractable dans le dispositif d'administration de médicament (1).

2. Unité d'aiguille (2) selon la revendication 1,
dans laquelle la position de départ de l'aiguille (5) est la position dans laquelle l'aiguille (5) est exposée et la position d'extrémité est la position dans laquelle l'aiguille (5) est rétractée.

3. Unité d'aiguille (2) selon l'une quelconque des revendications précédentes, dans laquelle le dispositif de retenue d'aiguille (3) est fixé dans sa position par rapport à un boîtier (7).

4. Unité d'aiguille (2) selon l'une quelconque des revendications précédentes, dans laquelle le dispositif de retenue d'aiguille (3) est configuré pour libérer l'aiguille (5) d'un état verrouillé dans un état déverrouillé.

5. Unité d'aiguille (2) selon la revendication 4,
dans laquelle les moyens de rétraction (12) sont configurés pour rétracter l'aiguille (5) lorsque l'aiguille (5) est déverrouillée.

6. Unité d'aiguille (2) selon l'une quelconque des revendications précédentes, dans laquelle l'aiguille (5), qui est positionnée dans la position de départ, est configurée pour forcer l'organe d'arrêt (6) dans une direction radiale vers l'extérieur par rapport à ladite aiguille (5).

7. Unité d'aiguille (2) selon l'une quelconque des revendications précédentes, dans laquelle l'organe d'arrêt (6) comprend une charnière.

8. Unité d'aiguille (2) selon l'une quelconque des revendications précédentes, dans laquelle l'organe d'arrêt (6) est configuré pour permettre un mouvement de l'aiguille (5) dans la direction distale par rapport à l'ouverture (14) jusque dans la position de départ tout en mettant en prise l'aiguille (5) avec ledit dispositif d'administration de médicament (1).

9. Unité d'aiguille (2) selon l'une quelconque des revendications 1 à 8,
dans laquelle l'organe d'arrêt (6) comprend une pince.

10. Dispositif d'administration de médicament (1) comprenant une unité d'aiguille (2) selon l'une quelconque des revendications précédentes et comprenant en outre un boîtier (7), le dispositif de retenue d'aiguille (3), les moyens de rétraction d'aiguille (12) et l'ensemble d'aiguille (5, 16) comprenant une aiguille (5), l'ensemble d'aiguille (5, 16) étant rétractable dans le boîtier (7) d'une position de départ, dans laquelle l'aiguille (5) de l'ensemble d'aiguille (5, 16) est exposée, jusqu'à une position d'extrémité, dans laquelle l'aiguille (5) n'est pas exposée, et le dispositif de retenue d'aiguille (3) étant configuré pour libérer l'ensemble d'aiguille (5, 16) depuis un état initial verrouillé dans un état déverrouillé de telle sorte qu'il puisse être rétracté par le moyen de rétraction d'aiguille (12) et fournissant en outre l'organe d'arrêt (6) de telle sorte que lors du retrait de l'aiguille (5), une ouverture (14) à travers laquelle l'aiguille (5) fait saillie soit au moins en partie fermée pour empêcher une réexposition subséquente de l'aiguille (5).

11. Dispositif d'administration de médicament (1) selon la revendication 10, comprenant un organe d'éjection (15) configuré pour déverrouiller l'ensemble d'aiguille (5, 16).

12. Dispositif d'administration de médicament (1) selon l'une quelconque des revendications 10 ou 11,
comprenant un joint d'aiguille (11) placé à une extrémité proximale de l'unité d'aiguille (2).
